(19) Europäisches Patentamt

European Patent Office

Office européen des brevets

(11) **EP 4 776 292 A1**

(12) **EUROPEAN PATENT APPLICATION**

(43) Date of publication:
**15.07.2026 Bulletin 2026/29**

(21) Application number: **26150485.6**

(22) Date of filing: **07.01.2026**

(51) International Patent Classification (IPC):
**G16B 30/10** (2019.01)

(52) Cooperative Patent Classification (CPC):
**G16B 30/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **09.01.2025 JP 2025003791**

(71) Applicant: **FUJITSU LIMITED**
**Kawasaki-shi, Kanagawa 211-8588 (JP)**

(72) Inventors:
- **HARA, Nobuyuki**
  **Kawasaki-shi, 211-8588 (JP)**
- **HASHIMA, Masayoshi**
  **Kawasaki-shi, 211-8588 (JP)**
- **KIBUNE, Masaya**
  **Kawasaki-shi, 211-8588 (JP)**

(74) Representative: **Haseltine Lake Kempner LLP**
**Cheapside House**
**138 Cheapside**
**London EC2V 6BJ (GB)**

(54) **COMPUTER PROGRAM, DATA PROCESSING APPARATUS, AND DATA PROCESSING METHOD FOR SEQEUNCE ALIGNMENT**

(57) A processing unit groups a plurality of sequences to be aligned in a multiple sequence alignment problem into a plurality of groups, selects a representative sequence from each of the plurality of groups, and causes an Ising machine to perform a first alignment process among the representative sequences of the plurality of groups. The processing unit replaces the representative sequence before the first alignment process with the corresponding representative sequence obtained through the first alignment process in each of the plurality of groups, and causes the Ising machine to perform a second alignment process within each of the plurality of groups after the replacement of the representative sequences.

FIG. 1

EP 4 776 292 A1

## Description

FIELD

The embodiments discussed herein relate to a computer program, a data processing apparatus, and a data processing method.

BACKGROUND

A multiple sequence alignment method is a technique for aligning (sorting) a plurality of sequences each including a plurality of elements, according to the correspondences of elements between the sequences. The multiple sequence alignment method is used in various fields such as character recognition, analysis of time-series data such as audio and video data, and bioinformatics. For example, a multiple sequence alignment method may be used to detect, from a plurality of amino acid sequences, common domains that are functionally important, common domains that characterize the plurality of amino acid sequences, and others (see, for example, Takeshi Kawabata, "Multiple Sequence Alignment and its Application", [online], April 21, 2009, Nara Institute of Science and Technology, Structural and Functional Bioinformatics, [searched on October 15, 2024], Internet <URL: http://isw3.naist.jp/IS/Kawabata-lab/LECDOC_KIN DAI/2009/multi_09Apr21.pdf>).

Since problems that are solved by a multiple sequence alignment method (referred to as multiple sequence alignment problems) are non-deterministic polynomial (NP)-complete problems, a metaheuristic method such as simulated annealing (SA) is sometimes used as a computational technique. An Ising machine (also referred to as a Boltzmann machine) is an apparatus that executes such a metaheuristic method. See, for example, the following literatures.

Japanese Laid-open Patent Publication No. 2017-189176
U.S. Patent Application Publication No. 2013/0166218

In the case where a multiple sequence alignment problem is solved using an Ising machine, the number of variables included in an evaluation function for evaluating an alignment result increases as the number of sequences to be aligned and the length of each sequence increase. This may cause an increase in the time needed for a solution to converge, or may result in an inability to obtain a solution due to hardware limitations.

SUMMARY

In one aspect, it is desirable to reduce the number of variables used by an Ising machine in solving a multiple sequence alignment problem.

In one aspect, there is provided a computer program that causes a computer to perform a process including: grouping a plurality of sequences to be aligned in a multiple sequence alignment problem into a plurality of groups; selecting a representative sequence from each of the plurality of groups; causing an Ising machine to perform a first alignment process among representative sequences of the plurality of groups; replacing the representative sequence before the first alignment process with a corresponding representative sequence obtained through the first alignment process in each of the plurality of groups; and causing the Ising machine to perform a second alignment process within each of the plurality of groups after the replacing of the representative sequence.

BRIEF DESCRIPTION OF DRAWINGS

The invention is described, by way of example only, with reference to the following drawings, in which:

FIG. 1 illustrates an example of a data processing apparatus and a data processing method according to a first embodiment;
FIG. 2 illustrates an example of an alignment process;
FIG. 3 is a view for describing weight values;
FIG. 4 is a view for describing a gap insertion constraint;
FIG. 5 illustrates an example of the hardware of a data processing apparatus according to a second embodiment;
FIG. 6 is a block diagram illustrating an example of the functions of the data processing apparatus;
FIG. 7 is a flowchart illustrating an example procedure for a process performed by the data processing apparatus;
FIG. 8 illustrates an example of grouping sequences;
FIG. 9 illustrates an example of an alignment process on representative sequences;

FIG. 10 illustrates an example of a replacement process based on representative sequences obtained through the alignment process;

FIG. 11 illustrates an example of the alignment process performed for each group;

FIG. 12 is a flowchart illustrating an example procedure for determining the number of groups and performing grouping;

FIG. 13 illustrates an example of the alignment process performed on each partial sequence;

FIG. 14 is a flowchart illustrating an example procedure for grouping based on similarity between sequences;

FIG. 15 illustrates an example of selecting reference sequences;

FIG. 16 illustrates an example of grouping based on similarity between sequences;

FIG. 17 illustrates an example of a group in which the number of sequences exceeds $GS_{max}$; and

FIG. 18 illustrates an example of reassigning t selected non-reference sequences to other groups.

DESCRIPTION OF EMBODIMENTS

Hereinafter, embodiments of the present disclosure will be described with reference to the drawings.

(First Embodiment)

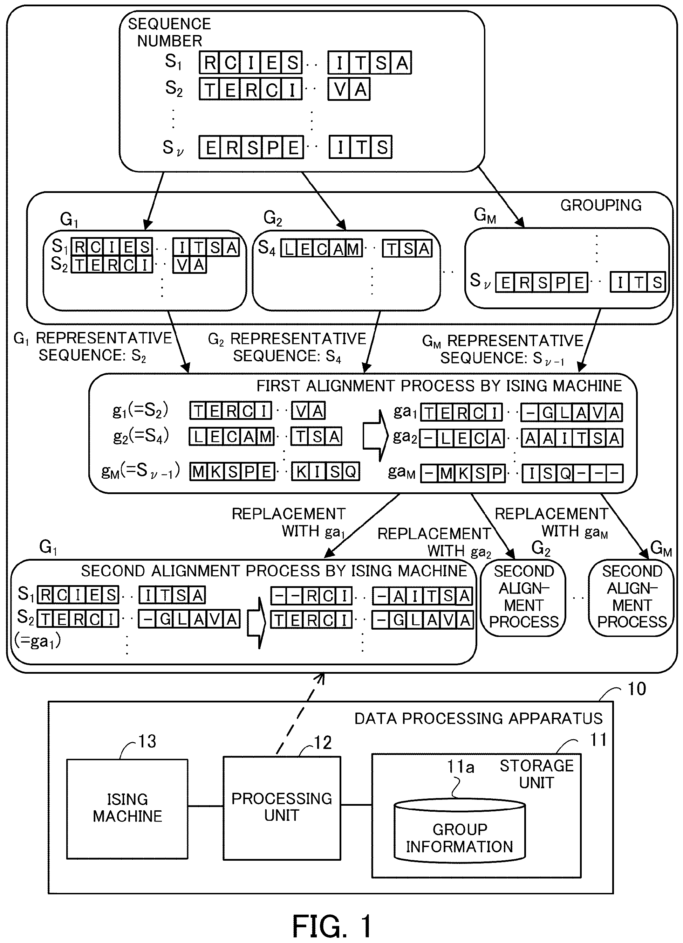

FIG. 1 illustrates an example of a data processing apparatus and a data processing method according to a first embodiment.

The data processing apparatus 10 according to the first embodiment includes a storage unit 11, a processing unit 12, and an Ising machine 13.

The storage unit 11 is a volatile storage device (for example, an electronic circuit such as a dynamic random access memory (DRAM)) or a non-volatile storage device (for example, an electronic circuit such as a flash memory or a hard disk drive (HDD)). The storage unit 11 may include an electronic circuit such as a register. The storage unit 11 stores group information 11a, which is information on sequences grouped in a manner that will be described later. The storage unit 11 may store, for example, problem information on a multiple sequence alignment problem input to the data processing apparatus 10 by a user. The problem information may include, in addition to data on a plurality of sequences to be aligned, information such as the degrees of similarity (substitution scores to be described later) between elements included in the sequences. In addition, the storage unit 11 may store various data such as computational conditions for the multiple sequence alignment problem. In this connection, examples of the sequences to be aligned include a plurality of amino acid sequences, a plurality of gene sequences, and time-series data such as character recognition data, audio data, and video data.

The processing unit 12 may be implemented by, for example, a processor that is hardware such as a central processing unit (CPU), a graphics processing unit (GPU), or a digital signal processor (DSP). The processor may include a plurality of processor cores. The processing unit 12 may include a plurality of processors. A set of processors may also be referred to as a multiprocessor or simply a "processor". The processor executes, for example, programs stored in a memory (which may be the storage unit 11) such as a RAM. In addition, the processing unit 12 may be implemented using an electronic circuit such as an application specific integrated circuit (ASIC) or a field programmable gate array (FPGA).

The processing unit 12 groups a plurality of sequences, which are to be aligned in the multiple sequence alignment problem, into a plurality of groups, and causes the Ising machine 13 to perform an alignment process in a manner that will be described later.

The Ising machine 13 is implemented using an electronic circuit such as an ASIC or an FPGA. Alternatively, the Ising machine 13 may be implemented by a processor, which is hardware such as a CPU, a GPU, or a DSP. The processor may include a plurality of processor cores. The Ising machine 13 may include a plurality of processors.

The Ising machine 13 searches for a sequence state that minimizes the value of the Ising-type evaluation function, using a Markov chain Monte Carlo method such as simulated annealing or replica exchange (also referred to as parallel tempering or the like). A state that produces the smallest value among the minimum values of the Ising-type evaluation function is taken as an optimal solution. By changing the signs of the Ising-type evaluation function, the Ising machine 13 is also able to search for a state that maximizes the value of the evaluation function. The sequence state may be represented by a combination of values of a plurality of variables included in the Ising-type evaluation function. Binary variables may be used as the variables.

Here, before describing the operation of the data processing apparatus 10, a reason for performing the alignment process using the Ising machine 13, instead of using a multidimensional dynamic programming method or a progressive method, which are examples of computational methods for multiple sequence alignment problems, will be described.

(Reason for Performing Alignment Process Using Ising Machine 13)

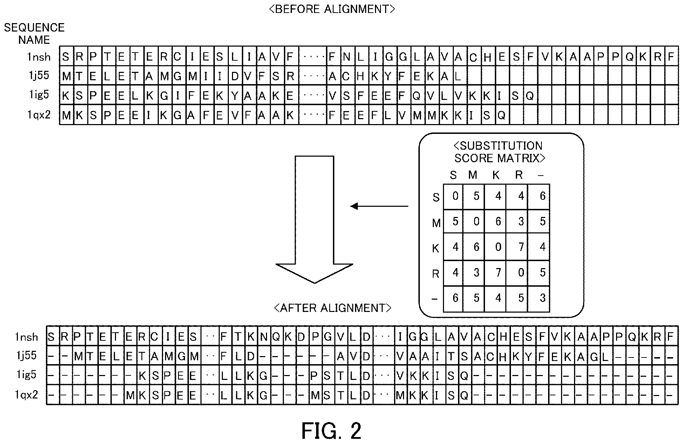

FIG. 2 illustrates an example of the alignment process. Illustrated in FIG. 2 is an example of performing the alignment process on four sequences (amino acid sequences) with sequence names 1nsh, 1j55, 1ig5, and 1qx2 using a multiple sequence alignment method. Each element (each letter in the figure) included in the sequences is an abbreviation representing one of 20 types of amino acids.

The correspondences between the elements are defined in advance as, for example, a substitution score matrix as illustrated in FIG. 2. A substitution score represents the degree of similarity between elements. A smaller substitution score between a pair of elements indicates a higher similarity therebetween. A pair of identical elements is given a substitution score of 0. Based on such substitution scores, the alignment process is performed so that as many identical or similar elements as possible belong to the same column. The alignment process is performed by inserting gaps ("-" in FIG. 2) to change the positions of elements. The swapping between elements, the addition of an element, and the deletion of an element are not performed. The substitution score matrix also includes substitution scores between each element and the gap.

The alignment result obtained through the above-described alignment process may be evaluated by using, for example, an evaluation function (sometimes referred to as "sum-of-pairs") expressed by the following Expression (1).

$$S(m) = \sum_{k<l} \sum_{i} S(m_i^k, m_i^l) \tag{1}$$

In Expression (1), $S(m_i^k, m_i^l)$ represents a substitution score for a pair of an element in the i-th column of a sequence k (k-th row) and an element in the i-th column of a sequence l (l-th row). For example, with respect to three sequences with the sequence names 1nsh, 1j55, and 1ig5 in FIG. 2, the value of the evaluation function for the first column is calculated as presented in the following Expression (2).

$$\begin{aligned} S(m_1) &= \sum_{k<l} S(m_1^k, m_1^l) \\ &= S(m_1^1, m_1^2) + S(m_1^1, m_1^3) + S(m_1^2, m_1^3) \\ &= S(\text{``S''}, \text{``M''}) + S(\text{``S''}, \text{``K''}) + S(\text{``M''}, \text{``K''}) \\ &= 5 + 4 + 6 = 15 \end{aligned} \tag{2}$$

As computational methods for multiple sequence alignment problems, there are a multidimensional dynamic programming method and a progressive method (see, for example, the following Reference Document 1).

Reference Document 1: Tatsuya Akutsu, "Bioinformatics (2) Basic Sequence Analysis", [online], 2019, Bioinformatics Center, Institute for Chemical Research, Kyoto University, [searched on October 17, 2024], Internet <URL: https://www.bic.kyoto-u.ac.jp/takutsu/members/takutsu/sysbioinfo2019b.pdf>

The multidimensional dynamic programming method needs a computation time proportional to $2^N n^N$ for N sequences each having n elements (sequence length = n). Therefore, as the number of sequences increases, the computation becomes more difficult. The progressive method is a technique that starts alignment with a pair of sequences having high similarity, using a neighbor joining method or the like, and then sequentially adds other sequences for the alignment based on the alignment result. Since the progressive method starts the alignment with highly similar sequences, the influence of partial (local) similarity may lead to a worse value of the evaluation function than an overall (global) alignment result.

To deal with these, the data processing apparatus 10 solves multiple sequence alignment problems using the Ising machine 13. In the case where a multiple sequence alignment problem is solved using the Ising machine 13, an overall (global) alignment is achieved by simultaneously performing an alignment operation on a plurality of sequences in real time, which produces an effect of reducing the influence of local similarity between sequences.

The following describes an Ising-type evaluation function for evaluating an alignment result of a multiple sequence alignment problem obtained by the alignment process using the Ising machine 13.

(Ising-Type Evaluation Function)

To solve a multiple sequence alignment problem using the Ising machine 13, a variable $x_{s,n,i}$ is used. $x_{s,n,i}$

indicates whether the element in the column n of the sequence s has been moved to the column i by the alignment process. In the following description, $x_{s,n,i} = 1$ indicates that the element in the column n of the sequence s has been moved to the column i by the alignment process, and $x_{s,n,i} = 0$ indicates that the element in the column n of the sequence s has not been moved to the column i by the alignment process. The sequence s is expressed as s = $\{S_1, S_2, S_3, ...\}$. For example, $S_1$ denotes the sequence number of the first sequence (first row). The column n in which an element included in the sequence s is located is expressed as n = $\{N_1, N_2, N_3, ...\}$. For example, $N_1$ denotes the column number of the first element (first column).

A series of binary variables $x_{s,n,i}$ arranged in order from the beginning of the sequence is represented as a variable vector $x_{s,n}$. For example, in the case where a sequence "M, T, E, L, E, ..." having a sequence number $S_2$ becomes "-, -, M, T, E, ... " as a result of the alignment process, a variable vector for the element "M" having the column number $N_1$ changes from {1, 0, 0, 0, ...} to {0, 0, 1, 0, ...}.

The Ising-type evaluation function for evaluating an alignment result of the multiple sequence alignment problem is expressed as the sum of an objective function S(x), which is represented below, and constraint terms. The data processing apparatus 10 solves the multiple sequence alignment problem as, for example, a combinatorial optimization problem that searches for a combination of values of variables that minimizes the value of an evaluation function. The objective function S(x) is expressed as the following Expression (3) using binary variables.

$$S(x) = \sum_{s_q < s_t} \sum_{n_q} \sum_{n_t} \sum_{i} w_{s_q,n_q,s_t,n_t} x_{s_q,n_q,i} x_{s_t,n_t,i} \qquad (3)$$

In Expression (3), $x_{sq,nq,i}$ (where "q" is a subscript of "s" or "n") denotes a variable (binary variable) indicating that the element in the column $n_g$ of the sequence $s_q$ that is a reference sequence to be compared is in the column i after the alignment process. Similarly, $x_{st,nt,i}$ (where "t" is a subscript of "s" or "n") denotes a variable (binary variable) indicating that the element in the column nt of the sequence st that is a target sequence to be compared with) is in the column i after the alignment process.

$w_{sq,nq,st,nt}$ (where "q" and "t" are subscripts of "s" or "n") denotes a weight value between the element in the column $n_q$ of the comparison-reference sequence $s_q$ and the element in the column nt of the comparison-target sequence st. The weight value is a constant defined in advance in a weight matrix. As the weight matrix, for example, a substitution score matrix (a matrix representing the degrees of similarity between elements) as illustrated in FIG. 2 may be used. In the case where an element exists in the column of the comparison target, the weight value between these elements is introduced in S(x).

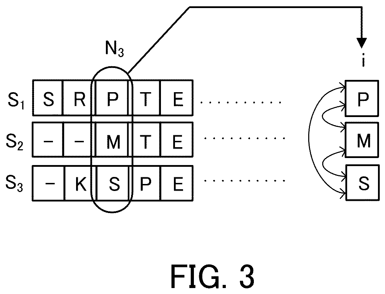

FIG. 3 is a view for describing weight values. Three sequences are illustrated in FIG. 3.

It is assumed that, as the element in the column $n_g$ of the comparison-reference sequence $s_q$, the element "P" in the column $N_3$ of the sequence $s_1$ is moved to the column i by the alignment process, and that, as the element in the column nt of the comparison-target sequence st, the element "M" or "S" in the column $N_3$ of the sequence $S_2$ or the sequence $s_3$ is moved to the column i by the alignment process. In this case, the weight value between the element "P" and the element "M" or "S" is introduced in S(x).

Similarly, it is assumed that, as the element in the column $n_q$ of the comparison-reference sequence $s_q$, the element "M" in the column $N_3$ of the sequence $s_2$ is moved to the column i by the alignment process, and that, as the element in the column nt of the comparison-target sequence st, the element "P" or "S" in the column $N_3$ of the sequence $s_1$ or the sequence $s_3$ is moved to the column i by the alignment process. In this case, the weight value between the element "M" and the element "P" or "S" is introduced in S(x).

The constraint terms included in the Ising-type evaluation function include a constraint term related to a gap insertion constraint, a constraint term related to a one-hot constraint on a column in which an element exists, a constraint term related to a one-hot constraint on columns in which a plurality of elements exist, and a constraint term related to an order constraint within a sequence.

The gap insertion constraint is a constraint for preventing excessive insertion of gaps during the alignment process. The constraint term related to the gap insertion constraint is expressed by the following Expression (4) using $x_{sq,nq,i}$ and $x_{st,nt,i}$ described above.

$$g \sum_{s_q} \sum_{s_t} \sum_{n_q} \sum_{i} x_{s_q,n_q,i} \left( 1 - \sum_{n_t} x_{s_t,n_t,i} \right) \qquad (4)$$

In Expression (4), g is a predefined fixed value representing a weight at the time of gap insertion.

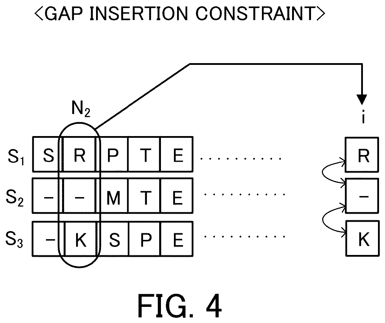

FIG. 4 is a view for describing the gap insertion constraint. Three sequences are illustrated in FIG. 4.

In the case where the element "R" in the column $N_2$ of the sequence $s_1$, as the element in the column $n_q$ of the comparison-reference sequence $s_q$, is moved to the column i by the alignment process and a gap is inserted in the column i of the sequence $s_2$ serving as the comparison-target sequence st, the constraint term increases in the positive direction based on Expression (4).

Similarly, in the case where the element "K" in the column $N_2$ of the sequence $S_3$, as the element in the column $n_q$ of the comparison-reference sequence $s_q$, is moved to the column i by the alignment process and a gap is inserted in the column i of the sequence $S_2$ serving as the comparison-target sequence st, the constraint term increases in the positive direction based on Expression (4).

Although the insertion of a gap may reduce the overall value of the evaluation function, such a constraint term is used to prevent excessive insertion of gaps.

The one-hot constraint on a column in which an element exists is a constraint that each element in a sequence exists only in any one column. In other words, this constraint prevents a certain element from existing in a plurality of columns or in no column at all. For example, this constraint ensures that the element "E" in the column with the column number $N_1$ included in a sequence "E, T, S, P, E, ..." exists only in any one column of a sequence such as in "-, -, E, T, S, ..." after the alignment process. With respect to each element, the sum of the numerical values of a variable vector always equals one (one-hot constraint). In this case, the variable vector for the element "E" with the column number $N_1$ changes from {1, 0, 0, 0, ...} to {0, 0, 1, 0, ...}.

The constraint term related to the one-hot constraint on a column in which an element exists is expressed by the following Expression (5) using $x_{s,n,i}$ described above.

$$h_1 \sum_s \sum_n \left( 1 - \sum_i x_{s,n,i} \right)^2 \qquad (5)$$

In Expression (5), $h_1$ is a predefined fixed value representing a weight for the one-hot constraint on a column in which an element exists.

The one-hot constraint on columns in which a plurality of elements exist is a constraint that a plurality of elements in the same sequence do not exist in the same column. That is, this constraint prevents a plurality of elements from existing in a certain column in the same sequence. For example, in a sequence "-, -, E, T, S, ...," a variable vector for the element "E" in the column with the column number $N_3$ is represented as {0, 0, 1, 0, 0, ...}, and a variable vector for the element "T" in the column with the column number $N_4$ is represented as {0, 0, 0, 1, 0, ...}. A variable vector for the element "S" in the column with the column number $N_5$ is represented as {0, 0, 0, 0, 1, ...}. In this way, the arrangement in which the plurality of elements "E", "T," and "S" exist in different columns within the same sequence satisfy this constraint.

The constraint term related to the one-hot constraint on columns in which a plurality of elements exist is expressed by the following Expression (6) using $x_{s,n,i}$ described above.

$$h_2 \sum_s \sum_n \left\{ \left( \sum_i x_{s,n,i} - 1 \right)^2 + \left( \sum_i x_{s,n,i} - 0 \right)^2 - 1 \right\} \qquad (6)$$

In Expression (6), $h_2$ is a predefined fixed value representing a weight for the one-hot constraint on columns in which a plurality of elements exist. Expression (6) expresses a constraint that each element in the same sequence exists in a different column, in other words, a constraint that a plurality of elements do not exist in the same column in one sequence. Therefore, with respect to each column, the sum of the numerical values of the binary variables corresponding to all elements of a sequence s always equals one, provided that the element in that column is not a gap (one-hot constraint).

The order constraint within a sequence ensures that the order of elements within the sequence is maintained (column numbers are not swapped) even after the alignment process. The constraint term related to the order constraint is expressed by the following Expression (7).

$$o \sum_s \sum_n \sum_{i \leq j} x_{s,n,j} x_{s,n+1,i} \qquad (7)$$

In Expression (7), $x_{s,n,j}$ is a variable (binary variable) indicating that the element in the column n of the sequence s is in the column j after the alignment process. $x_{s,n+1,i}$ is a variable (binary variable) indicating that the element in the column n+1 of the sequence s is in the column i after the alignment process. o is a predefined fixed value representing a weight for the order constraint.

The order constraint, which is expressed by the constraint term of Expression (7), prevents a situation in which, when the element existing in the column n+1 of the sequence s is moved to the column i by the alignment process, the element in the column n of the same sequence is moved to a column j preceding the column i.

In the case where the Ising machine 13 searches for a solution to a multiple sequence alignment problem according to the above-described Ising-type evaluation function, the number of variables increases as the number of sequences and the length of each sequence increase. As the number of variables increases, the time needed for the solution to converge becomes long (that is, the processing time until a solution is obtained becomes long). Alternatively, the Ising machine 13 may fail to solve the problem due to its hardware limitations. For example, in the case where the upper limit of the number of variables that the Ising machine 13 is able to process is $10^5$ and the length (the number of elements) of each sequence is 100, the upper limit for the number of sequences is 10.

Therefore, the data processing apparatus 10 of the first embodiment performs the following processing (data processing) in order to reduce the number of variables used by the Ising machine 13 in solving a multiple sequence alignment problem.

(Data Processing Performed by Data Processing Apparatus 10)

The processing unit 12 of the data processing apparatus 10 groups a plurality of sequences to be aligned in a multiple sequence alignment problem, into a plurality of groups. In the example of FIG. 1, each sequence represents an amino acid sequence, and each element of the sequences is an abbreviation representing one of 20 types of amino acids.

FIG. 1 illustrates an example in which sequences with sequence numbers $S_1$ to $S_v$ are grouped into M groups with group numbers $G_1$ to $G_M$. For example, the processing unit 12 determines the number of groups M on the basis of the hardware limitations of the Ising machine 13. The processing unit 12 distributes v sequences, for example, randomly among M groups. The processing unit 12 may distribute the sequences so that similar sequences belong to the same group.

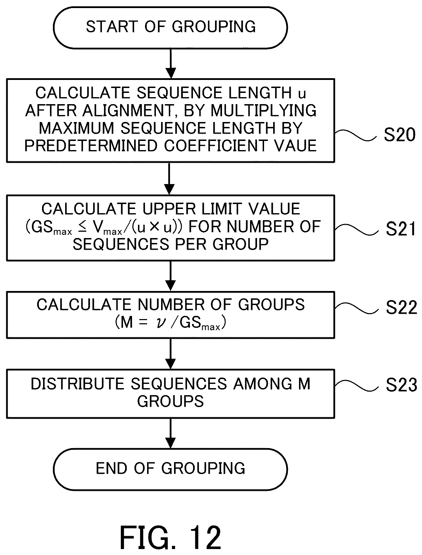

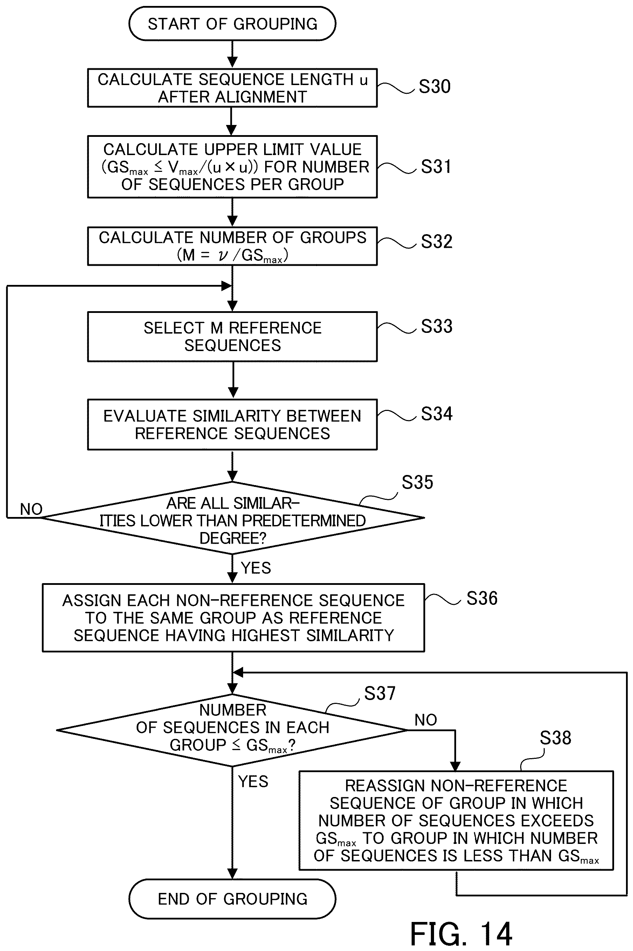

Specific example procedures for determining the number of groups M and distributing sequences among the groups will be described later (see FIGS. 12 and 14).

In the example of FIG. 1, the group with the group number $G_1$ includes the sequences with the sequence numbers $S_1$ and $S_2$. The group with the group number $G_2$ includes the sequence with the sequence number $S_4$. The group with the group number $G_M$ includes the sequence with the sequence number $S_v$. The result of the grouping is stored in the storage unit 11 as group information 11a.

After the grouping as described above, the processing unit 12 selects a representative sequence from each of the plurality of groups. For example, the processing unit 12 randomly selects a representative sequence from the plurality of sequences included in each group. In the following description, one representative sequence is selected for each group. However, in the case where the hardware limitations of the Ising machine 13 permit, a plurality of representative sequences may be selected from each group.

In the example of FIG. 1, the sequence with the sequence number $S_2$ is selected as a representative sequence from the group with the group number $G_1$, the sequence with the sequence number $S_4$ is selected as a representative sequence from the group with the group number $G_2$, and the sequence with the sequence number $S_{v-1}$ is selected as a representative sequence from the group with the group number $G_M$.

The processing unit 12 causes the Ising machine 13 to perform a first alignment process among the representative sequences of the plurality of groups.

FIG. 1 illustrates an example of the alignment process (first alignment process) performed on the M representative sequences with representative sequence numbers $g_1$ to $g_M$.

The Ising machine 13 searches for a sequence state that minimizes an Ising-type evaluation function represented as the sum of the objective function expressed by Expression (3) and the constraint terms presented in Expressions (4) to (7), for example, using a Markov chain Monte Carlo method such as simulated annealing.

For example, the Ising machine 13 selects one of variables $x_{sq,nq,i}$ included in the evaluation function, and determines whether to accept a flip of its value from 0 to 1 or from 1 to 0, based on a comparison result between the amount of change in the value of the evaluation function caused by the flip and a threshold. If the Ising machine 13 determines to accept the flip, the Ising machine 13 updates the value of the selected variable $x_{sq,nq,i}$. Such a process is iteratively performed until a predetermined termination condition is satisfied. For example, the termination condition is determined to be satisfied when the number of iterations of the above process reaches a predetermined number, or when the value of the evaluation function does not change (or the amount of change is within a predetermined range) even if the above process is iteratively performed a predetermined number of times.

In the case where the simulated annealing is employed, the threshold is determined based on a temperature parameter value and a random number value, and even a flip that increases the value of the evaluation function is stochastically permitted. This is to prevent the solution from being trapped in a local solution. The temperature parameter value is controlled to gradually decrease according to a predetermined schedule.

FIG. 1 illustrates representative sequences with representative sequence numbers $ga_1$ to $ga_M$ obtained by performing the first alignment process on the M representative sequences with the representative sequence numbers $g_1$ to $g_M$. The processing unit 12 receives the representative sequences with the representative sequence numbers $ga_1$ to $ga_M$ as an alignment result from the Ising machine 13. The alignment result of the first alignment process may be stored in the storage unit 11.

Then, in each of the plurality of groups, the processing unit 12 replaces the representative sequence before the first alignment process with the corresponding representative sequence obtained through the first alignment process. Then, the processing unit 12 causes the Ising machine 13 to perform a second alignment process within each of the plurality of groups after the replacement of the representative sequences.

In the example illustrated in FIG. 1, the representative sequence with the representative sequence number $g_1$ (the sequence with the sequence number $S_2$) in the group with the group number $G_1$ is replaced with the representative sequence with the representative sequence number $ga_1$ obtained through the first alignment process, and then the second alignment process is performed within the group. Similarly, in the groups with the group numbers $G_2$ to $G_M$, the representative sequences with the representative sequence numbers $g_2$ to $g_M$ are replaced with the corresponding representative sequences with the representative sequence numbers $ga_2$ to $ga_M$ obtained through the first alignment process, and the second alignment process is performed within each of the groups.

Note that the Ising machine 13 performs the second alignment process within each group, in a manner similar to the first alignment process. For example, the second alignment process within each group is performed in the order of the group numbers $G_1$, $G_2$, ..., and $G_M$ or in a random order.

The processing unit 12 may output the result of the second alignment process as a computation result (solution search result) of the multiple sequence alignment problem. For example, the processing unit 12 may cause a display device (not illustrated) to display the computation result, or may transmit the computation result to another information processing apparatus via a network. Alternatively, the processing unit 12 may store the computation result in the storage unit 11.

As described above, the data processing apparatus 10 according to the first embodiment groups a plurality of sequences to be aligned in a multiple sequence alignment problem into a plurality of groups, and selects a representative sequence from each of the plurality of groups. Then, the data processing apparatus 10 causes the Ising machine 13 to perform the first alignment process among the representative sequences of the plurality of groups. Then, in each of the plurality of groups, the data processing apparatus 10 replaces the representative sequence before the first alignment process with the corresponding representative sequence obtained through the first alignment process. Then, the data processing apparatus 10 causes the Ising machine 13 to perform the second alignment process for each of the plurality of groups after the replacement of the representative sequences. With the above approach, the Ising machine 13 does not need to perform the alignment process on all the sequences to be aligned at a time. Thus, it is possible to reduce the number of variables used by the Ising machine 13 in solving the multiple sequence alignment problem (the number of variables used in one solving operation).

Since the number of variables is reduced as described above, it is possible to avoid an increase in the time needed for the solution to converge in the problem solving of the Ising machine 13. In addition, regardless of the hardware limitations of the Ising machine 13, it is possible to solve a larger scale of problems.

The data processing apparatus 10 that provides the above-described effects is expected to be useful as a means to shorten the time needed for drug discovery and exploration of new materials. For example, in light of the recent COVID pandemic, rapid drug discovery is important for the containment or mitigation of pandemic impacts.

(Second Embodiment)

Next, a second embodiment will be described.

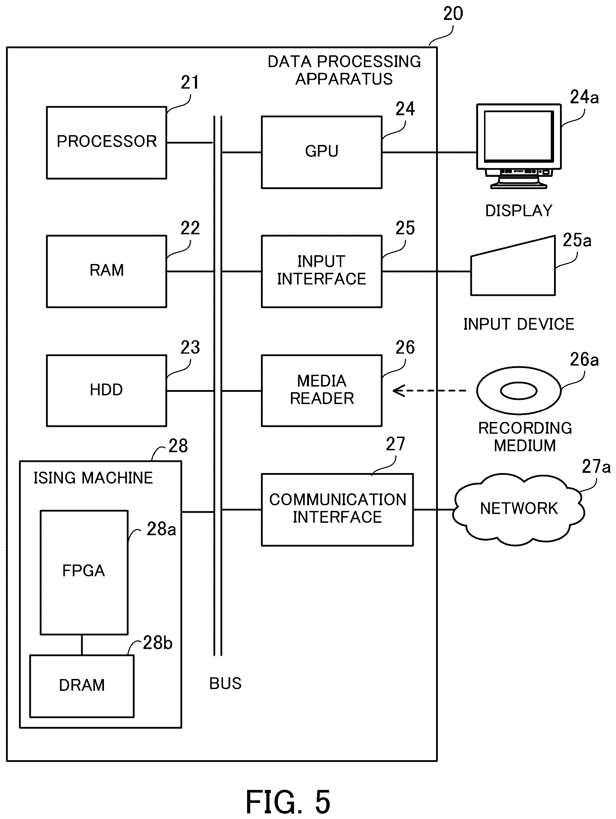

FIG. 5 illustrates an example of the hardware of a data processing apparatus according to the second embodiment.

The data processing apparatus 20 solves a multiple sequence alignment problem using an Ising machine 28. The data processing apparatus 20 may be referred to as a computer. The data processing apparatus 20 may be a client apparatus or a server apparatus.

The data processing apparatus 20 includes a processor 21, a RAM 22, an HDD 23, a GPU 24, an input interface 25, a media reader 26, a communication interface 27, and an Ising machine 28. These units are connected to a bus. The processor 21 corresponds to the processing unit 12 of the first embodiment. The RAM 22 or the HDD 23 corresponds to the storage unit 11 of the first embodiment. The Ising machine 28 corresponds to the Ising machine 13 of the first embodiment.

The processor 21 is a processor such as a GPU or a CPU including an arithmetic circuit that executes program instructions. The processor 21 loads at least a part of a program or data stored in the HDD 23 into the RAM 22 and executes the program. The processor 21 may include a plurality of processor cores. The data processing apparatus 20 may include a plurality of processors. Among a plurality of processes performed by the data processing apparatus 20, a certain process and another process may be performed by different processors. The processor may be referred to as processor circuitry. A set of a plurality of processors (multiprocessor) may be referred to as a "processor".

The RAM 22 is a volatile semiconductor memory that temporarily stores programs to be executed by the processor 21 and data to be used by the processor 21 during its operation. The data processing apparatus 20 may include a memory of a type other than the RAM 22, or may include a plurality of memories.

The HDD 23 is a non-volatile storage device that stores software programs such as an operating system (OS), middleware, and application software, and data. The programs include, for example, a program that causes the data processing apparatus 20 to solve a multiple sequence alignment problem. The data processing apparatus 20 may include another type of storage device such as a flash memory or a solid state drive (SSD), or may include a plurality of non-volatile storage devices.

The GPU 24 outputs images to a display 24a connected to the data processing apparatus 20 in accordance with instructions from the processor 21. As the display 24a, a cathode ray tube (CRT) display, a liquid crystal display (LCD), a plasma display panel (PDP), an organic electroluminescence (OEL) display, or the like may be used.

The input interface 25 receives input signals from an input device 25a connected to the data processing apparatus 20 and outputs the input signals to the processor 21. As the input device 25a, a pointing device such as a mouse, a touch panel, a touch pad, or a track ball, a keyboard, a remote controller, a button switch, or the like may be used. A plurality of types of input devices may be connected to the data processing apparatus 20.

The media reader 26 is a reading device that reads programs and data recorded on a recording medium 26a. As the recording medium 26a, for example, a magnetic disk, an optical disc, a magneto-optical disk (MO), a semiconductor memory, or another may be used. Magnetic disks include a flexible disk (FD) and an HDD. Optical discs include a compact disc (CD) and a digital versatile disc (DVD).

For example, the media reader 26 copies a program or data read from the recording medium 26a to another recording medium such as the RAM 22 or the HDD 23. The read program is executed by, for example, the processor 21. The recording medium 26a may be a portable recording medium, and may be used to distribute programs and data. The recording medium 26a and the HDD 23 may be referred to as computer-readable storage media.

The communication interface 27 is connected to a network 27a and communicates with other information processing apparatuses via the network 27a. The communication interface 27 may be a wired communication interface connected to a communication device such as a switch via a cable, or may be a wireless communication interface connected to a base station via a wireless link.

The Ising machine 28 performs an alignment process on a plurality of sequences by searching for a combination of variable values (representing an alignment state) that minimizes an Ising-type evaluation function, using a Markov chain Monte Carlo method such as simulated annealing. The Ising machine 28 is, for example, an accelerator card, and includes an FPGA 28a and a DRAM 28b. The alignment process is implemented by various circuits built in the FPGA 28a, and a memory in the FPGA 28a or the DRAM 28b. Alternatively, the Ising machine 28 may be implemented by one or more processors such as a GPU.

Next, the functions of the data processing apparatus 20 will be described.

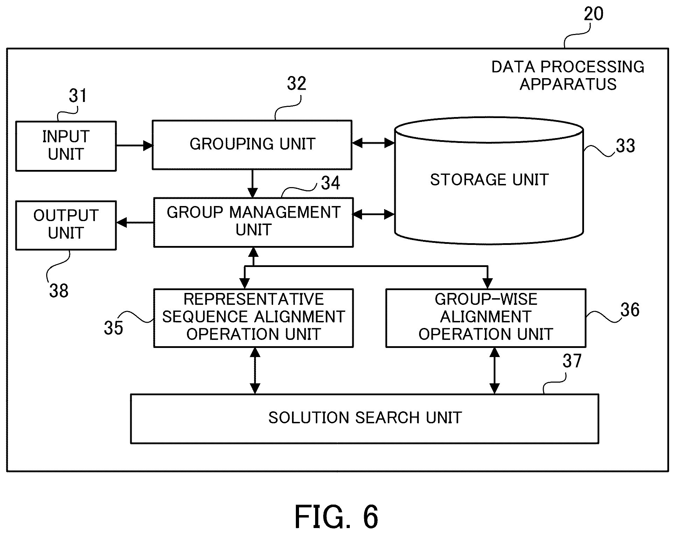

FIG. 6 is a block diagram illustrating an example of the functions of the data processing apparatus.

The data processing apparatus 20 includes an input unit 31, a grouping unit 32, a storage unit 33, a group management unit 34, a representative sequence alignment operation unit 35, a group-wise alignment operation unit 36, a solution search unit 37, and an output unit 38. These units implement functions similar to those of the storage unit 11, the processing unit 12, and the Ising machine 13 illustrated in FIG. 1.

The input unit 31, the grouping unit 32, the group management unit 34, the representative sequence alignment operation unit 35, the group-wise alignment operation unit 36, and the output unit 38 may be implemented by the processor 21 executing, for example, program modules. The storage unit 33 is implemented using a storage area secured in the RAM 22 or the HDD 23. The solution search unit 37 may be implemented by using the Ising machine 28.

The input unit 31 receives inputs such as problem information on a multiple sequence alignment problem and computational conditions. For example, the problem information includes, in addition to data on a plurality of sequences to be aligned, weight values included in the objective function of Expression (3), fixed values representing weights for the constraints included in Expressions (4) to (7), and others. These pieces of information may be input by the user operating the input device 25a or may be received from another computer via the network 27a.

The grouping unit 32 groups the plurality of sequences to be aligned, included in the input problem information, into a plurality of groups, and stores a result of the grouping in the storage unit 33 as group information.

The storage unit 33 stores the group information. In addition, the storage unit 33 may store the input problem information, computational conditions, alignment results, and others.

The group management unit 34 reads the group information from the storage unit 33, and selects a representative sequence from each of the plurality of groups. The group management unit 34 also acquires an alignment result via the representative sequence alignment operation unit 35, and stores the alignment result in the storage unit 33. Further, the group management unit 34 replaces, in the group information stored in the storage unit 33, the representative sequences before the alignment process with the corresponding representative sequences obtained through the alignment process. After the replacement, the group management unit 34 sends the sequences belonging to each group to the group-wise alignment operation unit 36, on a group-by-group basis. Then, the group management unit 34 acquires an alignment result for each group via the group-wise alignment operation unit 36, and stores the alignment results in the storage unit 33. Further, the group management unit 34 acquires the alignment results of all groups from the storage unit 33, and sends the alignment results to the output unit 38.

The representative sequence alignment operation unit 35 causes the solution search unit 37 (Ising machine 28) to perform the alignment process among the representative sequences of the respective groups.

The group-wise alignment operation unit 36 causes the solution search unit 37 (Ising machine 28) to perform the alignment process among the sequences included in each group.

The solution search unit 37 performs the alignment process on a plurality of sequences by searching for the alignment state that minimizes an Ising-type evaluation function, using a Markov chain Monte Carlo method such as simulated annealing.

The output unit 38 outputs an alignment result. The output unit 38 may cause the display 24a to display the alignment result, or may transmit the alignment result to another information processing apparatus via the network 27a. Alternatively, the output unit 38 may store the alignment result in the storage unit 33.

Next, a processing procedure performed by the data processing apparatus 20 will be described.

(Processing Procedure)

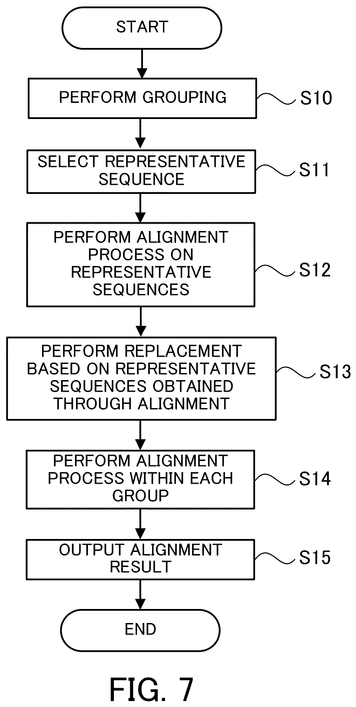

FIG. 7 is a flowchart illustrating an example procedure for a process performed by the data processing apparatus.

Step S10: The data processing apparatus 20 groups a plurality of sequences to be aligned, included in input problem information, into a plurality of groups. For example, the plurality of sequences to be aligned are a plurality of amino acid sequences, a plurality of gene sequences, or a plurality of time-series data such as character recognition data, audio data, and video data.

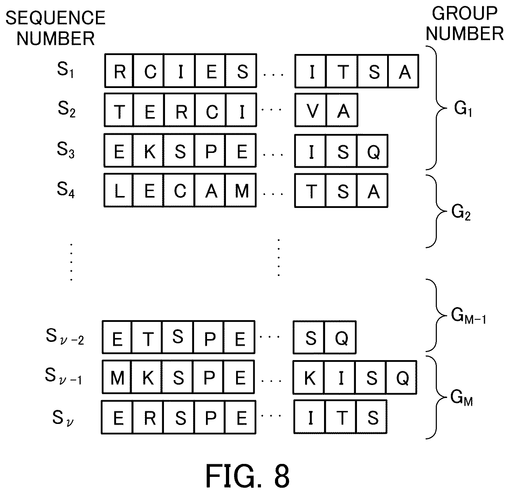

FIG. 8 illustrates an example of grouping sequences. In the example of FIG. 8, each sequence represents an amino acid sequence.

Sequences with sequence numbers $S_1$ to $S_v$ are grouped into M groups with group numbers $G_1$ to $G_M$. The group with the group number $G_1$ includes the sequences with the sequence numbers $S_1$ to $S_3$. The group with the group number $G_2$ includes the sequence with the sequence number $S_4$. The group with the group number $G_{M-1}$ includes the sequence with the sequence number $S_{v-2}$. The group with the group number $G_M$ includes the sequences with the sequence numbers $S_{v-1}$ and $S_v$. Specific example procedures for determining the number of groups M and performing grouping will be described later (see FIGS. 12 and 14).

Step S11: The data processing apparatus 20 selects a representative sequence from each of the plurality of groups. For example, in each of the M groups, the data processing apparatus 20 randomly selects one representative sequence from the plurality of sequences. In the following description, it is assumed that one representative sequence is selected for each group, but if the hardware limitations of the Ising machine 28 permit, a plurality of representative sequences may be selected.

Step S12: The Ising machine 28 of the data processing apparatus 20 performs the alignment process on the representative sequences. In the case where one representative sequence is selected for each of the M groups in step S11, the Ising machine 28 performs the alignment process by searching for an alignment state of the M representative sequences that minimizes the Ising-type evaluation function.

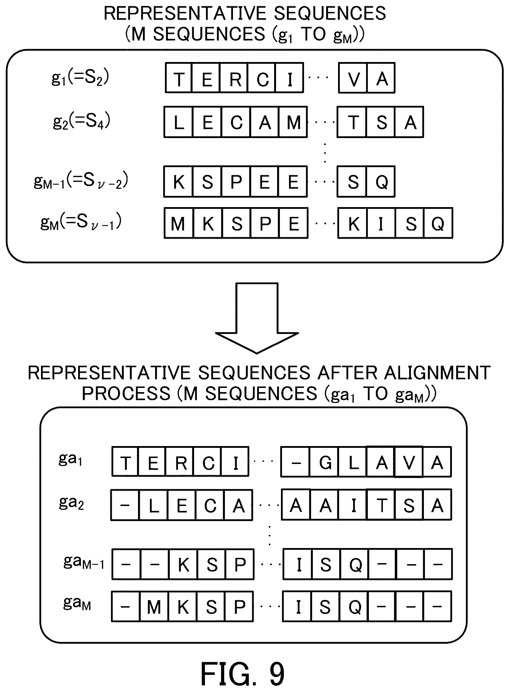

FIG. 9 illustrates an example of the alignment process on representative sequences. The sequences obtained by performing the alignment process on the M representative sequences with the representative sequence numbers $g_1$ to $g_M$ are depicted as representative sequences with representative sequence numbers $ga_1$ to $ga_M$.

The substitution score of sequences is defined as the sum, over all columns, of the substitution scores between elements belonging to the same column. In the alignment process, the column (position) to which each element belongs is determined such that the substitution score of the sequences is minimized. Specifically, gaps ("-" in FIG. 9) are inserted so that as many identical or similar elements as possible belong to the same column, thereby changing the column positions of elements during the alignment process.

For example, the representative sequences with the representative sequence numbers $ga_1$ and $ga_2$ are aligned such that the element "C" in the fourth column from the beginning and the element "A" in the last column match between the sequences. The representative sequences with the representative sequence numbers $ga_{M-1}$ and $ga_M$ are aligned such that the elements "K, S, P" in the third to fifth columns from the beginning and the elements "I, S, Q" in the sixth to fourth columns from the end match between the sequences.

Step S13: The data processing apparatus 20 replaces each representative sequence before the alignment process with the corresponding representative sequence obtained through the alignment process.

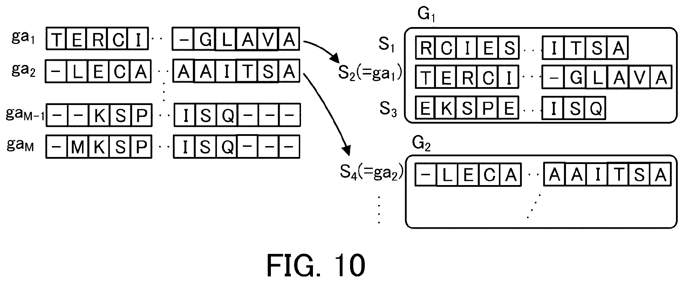

FIG. 10 illustrates an example of a replacement process based on representative sequences obtained through the alignment process.

As illustrated in FIG. 10, the representative sequence (sequence number $S_2$) of the group with the group number $G_1$ is replaced with the representative sequence with the representative sequence number $ga_1$ among the representative sequences with the representative sequence numbers $ga_1$ to $ga_M$ obtained through the alignment process. In addition, the representative sequence (sequence number $S_4$) of the group with the group number $G_2$ is replaced with the representative sequence with the representative sequence number $ga_2$. Similarly, in the other groups, the representative sequences before the alignment process are replaced with the corresponding representative sequences obtained through the alignment process.

Step S14: The Ising machine 28 of the data processing apparatus 20 performs the alignment process within each of the plurality of groups.

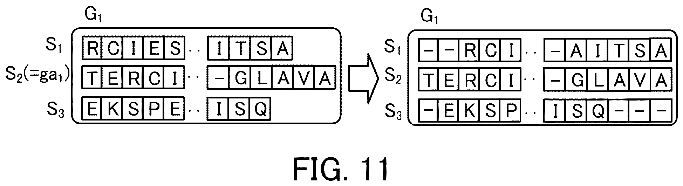

FIG. 11 illustrates an example of the alignment process performed for each group. FIG. 11 illustrates an example of the alignment process performed in the group with the group number $G_1$.

As described above, in the alignment process, the column (position) to which each element belongs is determined such that the substitution score of sequences is minimized. In the alignment process, gaps ("-" in FIG. 11) are inserted so that as many identical or similar elements as possible belong to the same column, thereby changing the column positions of elements.

In the example of FIG. 11, the sequences with the sequence numbers $S_1$ and $S_2$ are aligned such that the elements "R, C, I" in the third to fifth columns from the beginning and the element "A" in the last column match between the sequences. The sequences with the sequence numbers $S_2$ and $S_3$ are aligned such that the element "E" in the second column from the beginning matches between the sequences.

With respect to the groups with the group numbers $G_2$ to $G_M$, the alignment process is performed in the same manner. The alignment process may be performed for the groups, for example, in the order of the group numbers $G_1$, $G_2$, ..., and $G_M$, or in a random order.

Step S15: When the alignment process is completed for all the groups, the data processing apparatus 20 outputs the alignment result of all the sequences, thereby completing the process.

Next, a specific example procedure for determining the number of groups and performing the grouping will be described.

FIG. 12 is a flowchart illustrating an example procedure for determining the number of groups and performing grouping.

Step S20: The data processing apparatus 20 calculates the sequence length u after alignment. Specifically, the data processing apparatus 20 obtains the maximum sequence length of the plurality of sequences (all sequences before alignment) from input problem information, and calculates u by multiplying the maximum sequence length by a predetermined coefficient value.

Step S21: The data processing apparatus 20 calculates an upper limit value $GS_{max}$ for the number of sequences per group. The data processing apparatus 20 calculates $GS_{max}$ according to the following expression: $GS_{max} \leq V_{max}/(u \times u)$, where $V_{max}$ denotes an upper limit value for the number of variables included in the Ising-type evaluation function. $V_{max}$ is determined in advance based on the hardware limitations of the Ising machine 28, and is stored in the storage unit 33, for example.

Step S22: The data processing apparatus 20 calculates the number of groups M. The data processing apparatus 20 calculates M according to the following expression: $M = v/GS_{max}$, where v denotes the total number of sequences to be aligned.

Step S23: The data processing apparatus 20 distributes the sequences among M groups such that the number of sequences in each group is less than or equal to $GS_{max}$. For example, the data processing apparatus 20 randomly distributes the v sequences among the M groups.

Through the above process, the sequences are grouped into M groups.

The data processing apparatus 20 of the second embodiment is able to provide the same effects as those of the data processing apparatus 10 of the first embodiment. That is, the Ising machine 28 does not need to perform the alignment process on all sequences to be aligned at a time. That is, it is possible to reduce the number of variables used by the Ising machine 28 in solving a multiple sequence alignment problem (the number of variables used for one solving operation).

In addition, the data processing apparatus 20 calculates a second upper limit value ($GS_{max}$) for the number of sequences per group among the plurality of groups, based on the first upper limit value ($V_{max}$) for the number of variables included in the Ising-type evaluation function and the maximum sequence length of the plurality of sequences. Then, the data processing apparatus 20 calculates the number of groups M by dividing the number of sequences (total number of sequences v) by the second upper limit value. That is, the number of groups M may be determined according to, for example, $V_{max}$ that reflects the hardware limitations of the Ising machine.

Incidentally, there is a case where the number of sequences to be aligned is exceedingly large, and even after the grouping is performed, the number of variables may exceed $V_{max}$ due to the number of sequences and their sequence lengths. For example, in the case where the upper limit on the number of variables that the Ising machine 28 is able to handle is $10^5$ and the sequence length is 100, the upper limit for the number of sequences is 10.

Therefore, in the case where the sequence length is 100 and one representative sequence is selected per group, the maximum number of groups and the maximum number of sequences per group are both 10. In actual, there are large-scale problems in which the number of sequences exceeds 1000 and the sequence length exceeds 100. In such problems, even grouping may fail to reduce the number of variables used in one solving operation to $V_{max}$ or less.

In such cases, in one or both of steps S12 and S14 in FIG. 7, the data processing apparatus 20 may divide the sequences to be aligned into a plurality of partial sequences in the sequence direction (column direction) of the elements so that each partial sequence has a shorter sequence length, and then perform the alignment process for each partial sequence. Hereinafter, an example of sequence division and alignment process on partial sequences will be described.

(Examples of Sequence Division and Alignment Process on Partial Sequences)

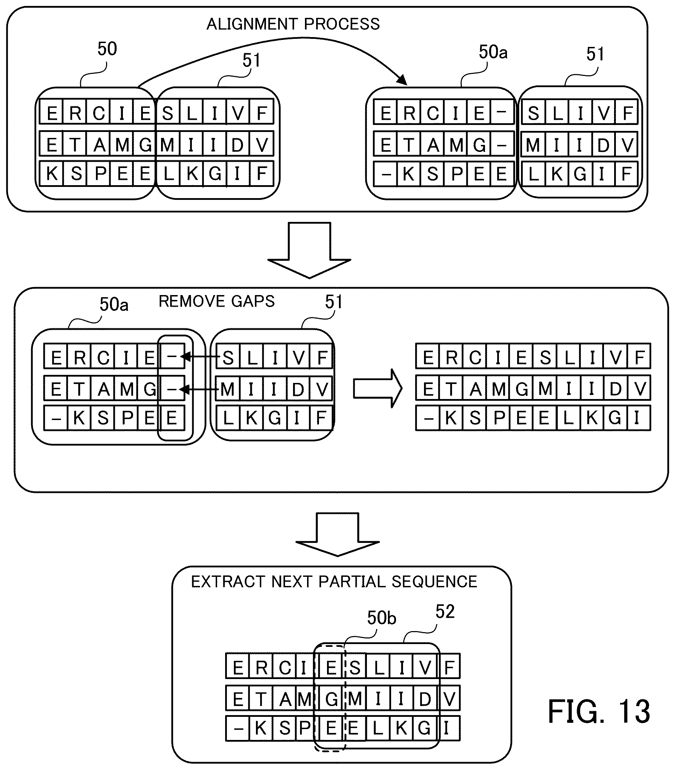

FIG. 13 illustrates an example of the alignment process performed on each partial sequence. FIG. 13 illustrates an example in which, with respect to three sequences, their first five columns from the beginning are extracted as a first partial sequence 50, and the alignment process is performed on the first partial sequence 50. The three sequences serve as representative sequences in step S12, and also serve as three sequences belonging to a certain group in step S14.

In the example of FIG. 13, as a result of the first alignment process or the second alignment process performed on the first partial sequence 50, gaps appear at the end of the first partial sequence 50a after the alignment process. Since the gaps are generated by the division, it is preferable to remove the gaps. To this end, the data processing apparatus 20 shifts a second partial sequence 51 following the first partial sequence 50a by one element, thereby removing the gaps at the end of the first partial sequence 50a. In the example of FIG. 13, the gaps are filled and removed by the first elements "S" and "M" of the second partial sequence.

Thereafter, the alignment process is performed on a third partial sequence 52 starting from the end 50b of the first partial sequence 50a from which the gaps have been removed.

By performing the alignment process for each partial sequence as described above, the Ising machine 28 is able to solve a large-scale multiple sequence alignment problem.

(Example of Grouping Based on Similarity Between Sequences)

In the grouping described with reference to FIG. 12, sequences are randomly distributed among M groups, as an example. Alternatively, the sequences may be distributed such that sequences having high similarity belong to the same group.

FIG. 14 is a flowchart illustrating an example procedure for grouping based on similarity between sequences. Steps S30 to S32 are executed in the same manner as steps S20 to S22 illustrated in FIG. 12.

Step S33: The data processing apparatus 20 selects, from the plurality of sequences (all sequences) to be aligned, one or more first sequences (hereinafter, referred to as reference sequences) the number of which corresponds to the number of groups M. For example, the data processing apparatus 20 randomly selects M reference sequences from all the sequences.

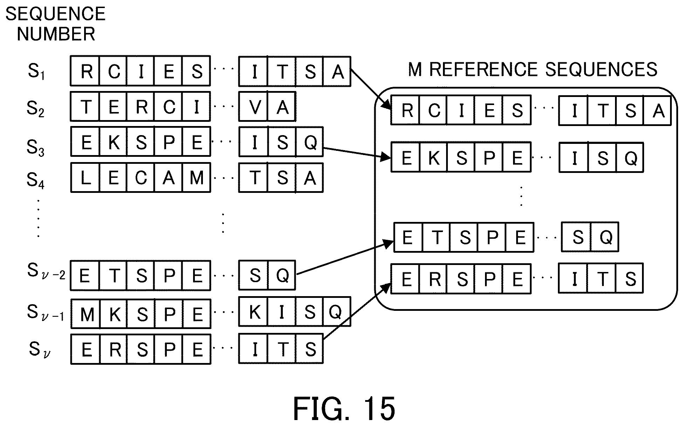

FIG. 15 illustrates an example of selecting reference sequences. In the example of FIG. 15, M reference sequences including sequences with sequence numbers $S_1$, $S_3$, $S_{v-2}$, and $S_v$ are selected from v sequences.

Step S34: The data processing apparatus 20 evaluates the similarity between the reference sequences. The data processing apparatus 20 evaluates the similarity, for example, by calculating the degree of similarity for all combinations between the reference sequences. The degree of similarity between the reference sequences is calculated based on the degrees of similarity between elements located in each corresponding column. The degree of similarity between reference sequences is calculated, for example, as follows.

First, the data processing apparatus 20 selects two reference sequences from the M selected reference sequences. Then, for each corresponding position (column) from the first column to the last column, the data processing apparatus 20 calculates the degree of similarity between the corresponding elements of the two selected reference sequences. The data processing apparatus then sums the degrees of similarity, thereby obtaining the degree of similarity between the two reference sequences.

The substitution scores illustrated in FIG. 2 may be used as the degrees of similarity between elements. The substitution scores are input as a part of problem information to and stored in the data processing apparatus 20. In the case where the substitution scores as illustrated in FIG. 2 are used as the degrees of similarity, a smaller value regarding the degree of similarity between two reference sequences indicates higher similarity therebetween.

Such calculation of the degree of similarity between reference sequences is performed for all combinations of the M reference sequences.

Step S35: The data processing apparatus 20 determines whether the similarities between the reference sequences are all lower than a predetermined degree. In the case where the degrees of similarity calculated using the substitution scores as described above are used for the similarity evaluation, the data processing apparatus 20 determines that the similarity is lower than the predetermined degree if the degree of similarity is greater than or equal to a predetermined value.

If the data processing apparatus 20 determines that the similarities between the reference sequences are all lower than the predetermined degree, the data processing apparatus 20 executes step S36. If the data processing apparatus 20 determines that any of the similarities between the reference sequences is not lower than the predetermined degree, the data processing apparatus 20 repeats step S33 and subsequent steps.

It is preferable that the similarity between sequences belonging to different groups is low, so that the alignment result is not affected by local similarity. If the similarity between reference sequences is high, the similarity between sequences belonging to different groups may become high. In order to avoid this situation, the above determination is performed.

Step S36: The data processing apparatus 20 assigns, among the plurality of sequences (all sequences), each second sequence (hereinafter, referred to as non-reference sequences) other than the reference sequences, into the same group as a reference sequence having the highest similarity thereto.

For example, the data processing apparatus 20 selects one of the non-reference sequences and calculates the degree of similarity to each of the M reference sequences, to evaluate the similarity. The degree of similarity may be calculated in the same manner as in step S34. Then, the data processing apparatus 20 assigns the selected non-reference sequence into the same group as the reference sequence having the highest similarity (the smallest value regarding the degree of similarity).

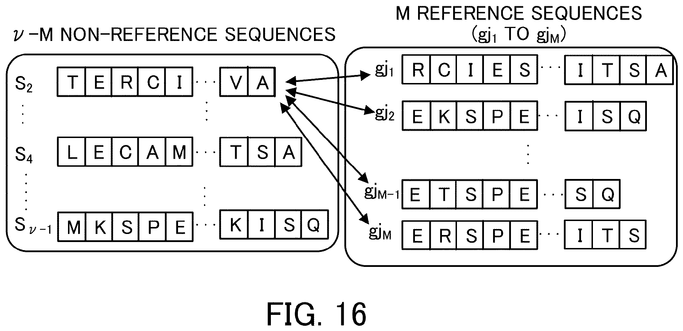

FIG. 16 illustrates an example of grouping based on similarity between sequences.

For example, in the case where the sequence with the sequence number $S_2$ is selected from v-M non-reference sequences, the similarity between the sequence and each of the M reference sequences ($gj_1$ to $gj_M$) is evaluated. For example, in the case where the sequence with the sequence number $S_2$ has the highest similarity to the reference sequence $gj_1$, the sequence is assigned to the same group as the reference sequence $gj_1$. The above process is also performed on all the other non-reference sequences.

Step S37: The data processing apparatus 20 determines whether the number of sequences in each group is less than or equal to $GS_{max}$. If it is determined that the number of sequences in each group is less than or equal to $GS_{max}$ for all groups, the data processing apparatus 20 completes the grouping process. If it is determined that the number of sequences in any of the groups exceeds $GS_{max}$, the data processing apparatus 20 executes step S38.

Step S38: With respect to each group in which the number of sequences exceeds $GS_{max}$, the data processing apparatus 20 reassigns a non-reference sequence from that group to any group in which the number of sequences is less than $GS_{max}$. In step S38, the data processing apparatus 20 selects, from a group in which the number of sequences exceeds $GS_{max}$, a sequence having low similarity to the reference sequence of that group, and reassigns the selected sequence to a group in which the number of sequences is less than $GS_{max}$.

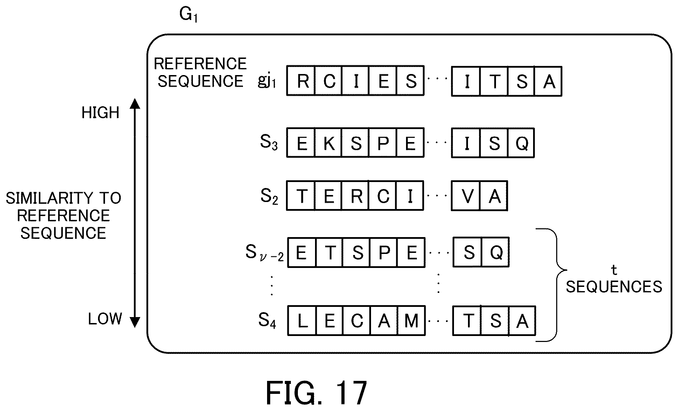

FIG. 17 illustrates an example of a group in which the number of sequences exceeds $GS_{max}$.

FIG. 17 illustrates an example in which the group with the group number $G_1$ includes t more sequences than $GS_{max}$. The data processing apparatus 20 sorts the non-reference sequences included in the group with the group number $G_1$ in order of similarity to the reference sequence $gj_1$. Then, the data processing apparatus 20 selects t non-reference sequences in ascending order of similarity, and reassigns the selected non-reference sequences to other groups.

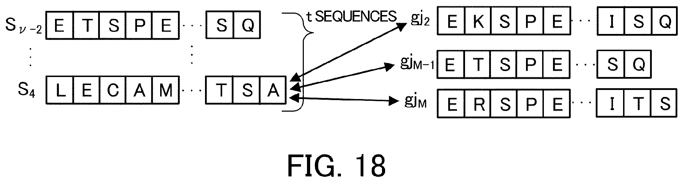

FIG. 18 illustrates an example of reassigning t selected non-reference sequences to other groups.

In the case where there are a plurality of groups in which the number of sequences is less than $GS_{max}$, the data processing apparatus 20 reassigns a target sequence to a group that includes a reference sequence having the highest similarity to that target sequence. As illustrated in FIG. 18, for example, in determining a destination group for the sequence with the sequence number $S_4$ among the t non-reference sequences, the data processing apparatus 20 evaluates the similarity between the sequence with the sequence number $S_4$ and each of the reference sequences $gj_2$, $gj_{M-1}$, and $gj_M$ belonging to the groups in which the number of sequences is less than $GS_{max}$.

In the case where the similarity between the sequence with the sequence number $S_4$ and the reference sequence $gj_2$ is the highest, the sequence with the sequence number $S_4$ is reassigned to the group to which the reference sequence $gj_2$ belongs.

After step S38, step S37 and subsequent steps are repeated.

The above grouping process uses the similarities between sequences, as in a progressive method, but generates groups such that the similarities between different groups are low. Therefore, representative sequences selected from the groups have low similarities therebetween, and thus the alignment result is less likely to be affected by local similarity.

The order of the steps in FIG. 14 is merely an example, and the order of the steps may be changed as appropriate.

The processing contents of the data processing apparatus 20 according to the second embodiment have been described above. As described earlier, the processing contents may be implemented by causing the data processing apparatus 20 to execute a program.

The program may be recorded on a computer-readable recording medium (for example, the recording medium 26a). Examples of the recording medium include a magnetic disk, an optical disc, a magneto-optical disk, a semiconductor memory, and others. Magnetic disks include an FD and an HDD. Optical discs include a CD, a CD-recordable (CD-R), CD-rewritable (CD-RW), a DVD, and a DVD-R/RW. The program may be recorded on portable recording media, which are then distributed. In this case, the program may be copied from a portable recording medium to another recording medium (for example, the HDD 23) and executed.

In the above description, a plurality of amino acid sequences are mainly used as a plurality of sequences to be aligned, but the present disclosure is not limited thereto. The plurality of sequences to be aligned may be a plurality of gene sequences. In this case, bases such as adenine (A), guanine (G), cytosine (C), thymine (T), and uracil (U) may be used as elements included in each sequence, instead of amino acids.

In addition, time-series data such as character recognition data, audio data, and video data are also be used as sequences to be aligned. Character recognition results, voice data, or the like per unit time (unit frame) may be used as elements forming each sequence. Sequences of time-series data are described in, for example, the following Reference Documents 2 and 3.

Reference Document 2: TTH Nguyen et al., "Survey of Post-OCR Processing Approaches," July 2021, ACM Computing Surveys, Vol. 54, No. 6, Article 124

Reference Document 3: Takamitsu Matsubara and Jun Morimoto, "Canonical Multiple Sequence Alignment for Multiple Time-Series Analysis", 2013, IEICE Transactions, Vol. J96-D No. 2, pp. 298-305

Reference Document 2 describes a method for improving character recognition accuracy in character recognition by aligning and comparing character strings that are recognition results obtained by a plurality of recognition methods.

Reference Document 3 proposes a method for robustly performing time-based alignment on time-series data such as human motion, speech, or brain activity, without being affected by spatial diversity.

Heretofore, the computer program, the data processing apparatus, and the data processing method of the present disclosure have been described based on the embodiments. Note that these are merely examples and are not limited to the above description.

In one aspect, it is possible to reduce the number of variables used by an Ising machine in solving a multiple sequence alignment problem.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. A computer program that causes a computer to perform a process comprising:

   grouping a plurality of sequences to be aligned in a multiple sequence alignment problem into a plurality of groups;
   selecting a representative sequence from each of the plurality of groups;
   causing an Ising machine to perform a first alignment process among representative sequences of the plurality of groups;
   replacing the representative sequence before the first alignment process with a corresponding representative sequence obtained through the first alignment process in each of the plurality of groups; and
   causing the Ising machine to perform a second alignment process within each of the plurality of groups after the replacing of the representative sequence.

2. The computer program according to claim 1, wherein the process further includes:

   calculating, based on a first upper limit value for a number of variables included in an Ising-type evaluation function of the multiple sequence alignment problem and a maximum sequence length of the plurality of sequences, a second upper limit value for a number of sequences within each of the plurality of groups; and

calculating a number of the plurality of groups by dividing a number of the plurality of sequences by the second upper limit value.

3. The computer program according to claim 2, wherein the first upper limit value is determined in advance based on a hardware limitation of the Ising machine.

4. The computer program according to any of the preceding claims, wherein the process further includes:

dividing a sequence to be aligned into a plurality of partial sequences in a sequence direction of elements, in the first alignment process or the second alignment process; and
causing the Ising machine to perform the first alignment process or the second alignment process for each of the plurality of partial sequences.

5. The computer program according to claim 4, wherein

the plurality of partial sequences includes a first partial sequence (50) and a second partial sequence (51) following the first partial sequence, and
the process further includes

in response to a gap being generated at an end of the first partial sequence as a result of performing the first alignment process or the second alignment process on the first partial sequence, removing the gap by shifting the second partial sequence by one element, and
causing the Ising machine to perform the first alignment process or the second alignment process on a third partial sequence (52) starting from the end of the first partial sequence from which the gap has been removed.

6. The computer program according to any of the preceding claims, wherein the grouping of the plurality of sequences into the plurality of groups includes

selecting, from the plurality of sequences, one or more first sequences in a number corresponding to a number of the plurality of groups, the one or more first sequences having lower similarity to each other than a predetermined degree, and
assigning each of second sequences other than the one or more first sequences among the plurality of sequences to a same group as one of the one or more first sequences having highest similarity to said each of the second sequences.

7. The computer program according to any of the preceding claims, wherein the plurality of sequences are a plurality of amino acid sequences, a plurality of gene sequences, or a plurality of time-series data.

8. A data processing apparatus comprising:

an Ising machine; and
processing means for:

grouping a plurality of sequences to be aligned in a multiple sequence alignment problem into a plurality of groups;
selecting a representative sequence from each of the plurality of groups;
causing an Ising machine to perform a first alignment process among representative sequences of the plurality of groups;
replacing the representative sequence before the first alignment process with a corresponding representative sequence obtained through the first alignment process in each of the plurality of groups; and
causing the Ising machine to perform a second alignment process within each of the plurality of groups after the replacing of the representative sequence.

9. A data processing method executed by a computer, the data processing method comprising:

grouping a plurality of sequences to be aligned in a multiple sequence alignment problem into a plurality of groups;
selecting a representative sequence from each of the plurality of groups;
causing an Ising machine to perform a first alignment process among representative sequences of the plurality of groups;

replacing the representative sequence before the first alignment process with a corresponding representative sequence obtained through the first alignment process in each of the plurality of groups; and

causing the Ising machine to perform a second alignment process within each of the plurality of groups after the replacing of the representative sequence.

FIG. 1

<BEFORE ALIGNMENT>

SEQUENCE NAME

| 1nsh | S R P T E T E R C I E S L I A V F · · · · F N L I G G L A V A C H E S F V K A A P P Q K R F |
| 1j55 | M T E L E T A M G M I I D V F S R · · · · A C H K Y F E K A L |
| 1ig5 | K S P E E L K G I F E K Y A A K E · · · V S F E E F Q V L V K K I S Q |
| 1qx2 | M K S P E E I K G A F E V F A A K · · · F E E F L V M M K K I S Q |

<SUBSTITUTION SCORE MATRIX>

|   | S | M | K | R | − |
|---|---|---|---|---|---|
| S | 0 | 5 | 4 | 4 | 6 |
| M | 5 | 0 | 6 | 3 | 5 |
| K | 4 | 6 | 0 | 7 | 4 |
| R | 4 | 3 | 7 | 0 | 5 |
| − | 6 | 5 | 4 | 5 | 3 |

<AFTER ALIGNMENT>

| 1nsh | S R P T E T E R C I E S · · F T K N Q K D P G V L D · · · I G G L A V A C H E S F V K A A P P Q K R F |
| 1j55 | − − M T E L E T A M G M · · F L D − − − − − A V D · · · V A A I T S A C H K Y F E K A G L − − − − − |
| 1ig5 | − − − − − − K S P E E · · L L K G − − − P S T L D · · · V K K I S Q − − − − − − − − − − − − − − − |
| 1qx2 | − − − − − M K S P E E · · L L K G − − − M S T L D · · M K K I S Q − − − − − − − − − − − − − − − |

FIG. 2

FIG. 3

⟨GAP INSERTION CONSTRAINT⟩

FIG. 4

FIG. 5

FIG. 6

START

PERFORM GROUPING — S10

SELECT REPRESENTATIVE SEQUENCE — S11

PERFORM ALIGNMENT PROCESS ON REPRESENTATIVE SEQUENCES — S12

PERFORM REPLACEMENT BASED ON REPRESENTATIVE SEQUENCES OBTAINED THROUGH ALIGNMENT — S13

PERFORM ALIGNMENT PROCESS WITHIN EACH GROUP — S14

OUTPUT ALIGNMENT RESULT — S15

END

FIG. 7

SEQUENCE
NUMBER

GROUP
NUMBER

$S_1$ | R | C | I | E | S | ... | I | T | S | A |

$S_2$ | T | E | R | C | I | ... | V | A |

$S_3$ | E | K | S | P | E | ... | I | S | Q |

$G_1$

$S_4$ | L | E | C | A | M | ... | T | S | A |

$G_2$

$G_{M-1}$

$S_{\nu-2}$ | E | T | S | P | E | ... | S | Q |

$S_{\nu-1}$ | M | K | S | P | E | ... | K | I | S | Q |

$G_M$

$S_{\nu}$ | E | R | S | P | E | ... | I | T | S |

FIG. 8

REPRESENTATIVE SEQUENCES
(M SEQUENCES ($g_1$ TO $g_M$))

$g_1(=S_2)$ | T | E | R | C | I | ⋯ | V | A |

$g_2(=S_4)$ | L | E | C | A | M | ⋯ | T | S | A |

$g_{M-1}(=S_{\nu-2})$ | K | S | P | E | E | ⋯ | S | Q |

$g_M(=S_{\nu-1})$ | M | K | S | P | E | ⋯ | K | I | S | Q |

REPRESENTATIVE SEQUENCES AFTER ALIGNMENT
PROCESS (M SEQUENCES ($ga_1$ TO $ga_M$))

$ga_1$ | T | E | R | C | I | ⋯ | – | G | L | A | V | A |

$ga_2$ | – | L | E | C | A | ⋯ | A | A | I | T | S | A |

$ga_{M-1}$ | – | – | K | S | P | ⋯ | I | S | Q | – | – | – |

$ga_M$ | – | M | K | S | P | ⋯ | I | S | Q | – | – | – |

FIG. 9

ga₁ $\boxed{T}\boxed{E}\boxed{R}\boxed{C}\boxed{I}$ ·· $\boxed{-}\boxed{G}\boxed{L}\boxed{A}\boxed{V}\boxed{A}$

ga₂ $\boxed{-}\boxed{L}\boxed{E}\boxed{C}\boxed{A}$ ·· $\boxed{A}\boxed{A}\boxed{I}\boxed{T}\boxed{S}\boxed{A}$

ga_{M-1} $\boxed{-}\boxed{-}\boxed{K}\boxed{S}\boxed{P}$ ·· $\boxed{I}\boxed{S}\boxed{Q}\boxed{-}\boxed{-}\boxed{-}$

ga_M $\boxed{-}\boxed{M}\boxed{K}\boxed{S}\boxed{P}$ ·· $\boxed{I}\boxed{S}\boxed{Q}\boxed{-}\boxed{-}\boxed{-}$

G₁

S₁ $\boxed{R}\boxed{C}\boxed{I}\boxed{E}\boxed{S}$ ·· $\boxed{I}\boxed{T}\boxed{S}\boxed{A}$

S₂(=ga₁) $\boxed{T}\boxed{E}\boxed{R}\boxed{C}\boxed{I}$ ·· $\boxed{-}\boxed{G}\boxed{L}\boxed{A}\boxed{V}\boxed{A}$

S₃ $\boxed{E}\boxed{K}\boxed{S}\boxed{P}\boxed{E}$ ·· $\boxed{I}\boxed{S}\boxed{Q}$

G₂

S₄(=ga₂) $\boxed{-}\boxed{L}\boxed{E}\boxed{C}\boxed{A}$ ·· $\boxed{A}\boxed{A}\boxed{I}\boxed{T}\boxed{S}\boxed{A}$

# FIG. 10

FIG. 11

START OF GROUPING

CALCULATE SEQUENCE LENGTH u AFTER ALIGNMENT, BY MULTIPLYING MAXIMUM SEQUENCE LENGTH BY PREDETERMINED COEFFICIENT VAUE — S20

CALCULATE UPPER LIMIT VALUE $(GS_{max} \leq V_{max}/(u \times u))$ FOR NUMBER OF SEQUENCES PER GROUP — S21

CALCULATE NUMBER OF GROUPS $(M = \nu /GS_{max})$ — S22

DISTRIBUTE SEQUENCES AMONG M GROUPS — S23

END OF GROUPING

# FIG. 12

ALIGNMENT PROCESS

50

| E | R | C | I | E |
| E | T | A | M | G |
| K | S | P | E | E |

51

| S | L | I | V | F |
| M | I | I | D | V |
| L | K | G | I | F |

50a

| E | R | C | I | E | - |
| E | T | A | M | G | - |
| - | K | S | P | E | E |

51

| S | L | I | V | F |
| M | I | I | D | V |
| L | K | G | I | F |

REMOVE GAPS

50a

| E | R | C | I | E | - |
| E | T | A | M | G | - |
| - | K | S | P | E | E |

51

| S | L | I | V | F |
| M | I | I | D | V |
| L | K | G | I | F |

| E | R | C | I | E | S | L | I | V | F |
| E | T | A | M | G | M | I | I | D | V |
| - | K | S | P | E | E | L | K | G | I |

EXTRACT NEXT PARTIAL SEQUENCE

50b   52

| E | R | C | I | E | S | L | I | V | F |
| E | T | A | M | G | M | I | I | D | V |
| - | K | S | P | E | E | L | K | G | I |

FIG. 13

$$\text{START OF GROUPING}$$

CALCULATE SEQUENCE LENGTH u AFTER ALIGNMENT — S30

CALCULATE UPPER LIMIT VALUE ($GS_{max} \leq V_{max}/(u \times u)$) FOR NUMBER OF SEQUENCES PER GROUP — S31

CALCULATE NUMBER OF GROUPS ($M = \nu /GS_{max}$) — S32

SELECT M REFERENCE SEQUENCES — S33

EVALUATE SIMILARITY BETWEEN REFERENCE SEQUENCES — S34

S35 — ARE ALL SIMILAR-ITIES LOWER THAN PREDETERMINED DEGREE? — NO / YES

ASSIGN EACH NON-REFERENCE SEQUENCE TO THE SAME GROUP AS REFERENCE SEQUENCE HAVING HIGHEST SIMILARITY — S36

S37 — NUMBER OF SEQUENCES IN EACH GROUP $\leq GS_{max}$? — YES / NO

S38 — REASSIGN NON-REFERENCE SEQUENCE OF GROUP IN WHICH NUMBER OF SEQUENCES EXCEEDS $GS_{max}$ TO GROUP IN WHICH NUMBER OF SEQUENCES IS LESS THAN $GS_{max}$

END OF GROUPING

FIG. 14

SEQUENCE
NUMBER

$S_1$ | R | C | I | E | S | $\cdots$ | I | T | S | A |

$S_2$ | T | E | R | C | I | $\cdots$ | V | A |

$S_3$ | E | K | S | P | E | $\cdots$ | I | S | Q |

$S_4$ | L | E | C | A | M | $\cdots$ | T | S | A |

$S_{\nu-2}$ | E | T | S | P | E | $\cdots$ | S | Q |

$S_{\nu-1}$ | M | K | S | P | E | $\cdots$ | K | I | S | Q |

$S_\nu$ | E | R | S | P | E | $\cdots$ | I | T | S |

M REFERENCE SEQUENCES

R | C | I | E | S | $\cdots$ | I | T | S | A |

E | K | S | P | E | $\cdots$ | I | S | Q |

E | T | S | P | E | $\cdots$ | S | Q |

E | R | S | P | E | $\cdots$ | I | T | S |

FIG. 15

31

M REFERENCE SEQUENCES
($gj_1$ TO $gj_M$)

$\nu$ -M NON-REFERENCE SEQUENCES

$S_2$  | T | E | R | C | I |···| V | A |

$S_4$  | L | E | C | A | M |···| T | S | A |

$S_{\nu-1}$  | M | K | S | P | E |···| K | I | S | Q |

$gj_1$  | R | C | I | E | S |···| I | T | S | A |

$gj_2$  | E | K | S | P | E |···| I | S | Q |

$gj_{M-1}$  | E | T | S | P | E |···| S | Q |

$gj_M$  | E | R | S | P | E |···| I | T | S |

FIG. 16

FIG. 17

$S_{\nu-2}$ | E | T | S | P | E | ··· | S | Q |

$S_4$ | L | E | C | A | M | ··· | T | S | A |

t SEQUENCES

$gj_2$ | E | K | S | P | E | ··· | I | S | Q |

$gj_{M-1}$ | E | T | S | P | E | ··· | S | Q |

$gj_M$ | E | R | S | P | E | ··· | I | T | S |

FIG. 18

## EUROPEAN SEARCH REPORT

**Application Number**

EP 26 15 0485

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MELODY LEE: "Modified Multiple Sequence Alignment Algorithm on Quantum Annealers (MAQ)", ARXIV.ORG CORNELL UNIVERSITY LIBRARY, 201 OLIN LIBRARY CORNELL UNIVERSITY ITHACA, NY 14853, [Online] 24 March 2024 (2024-03-24), XP091711951, DOI: 10.22369/ISSN.2153-4136/14/1/5 [retrieved on 2026-05-22] * whole document, in particular: p.4, par.2 * | 1-9 | INV. G16B30/10 |
| A | GIANNAKIS KONSTANTINOS ET AL: "A Quantum-inspired optimization Heuristic for the Multiple Sequence Alignment Problem in Bio-computing", 2019 10TH INTERNATIONAL CONFERENCE ON INFORMATION, INTELLIGENCE, SYSTEMS AND APPLICATIONS (IISA) IEEE, 15 July 2019 (2019-07-15), pages 1-8, XP033658945, DOI: 10.1109/IISA.2019.8900740 [retrieved on 2019-11-13] * the whole document * | 1-9 | |

**TECHNICAL FIELDS SEARCHED (IPC)**

G16B

The present search report has been drawn up for all claims

| Place of search | Date of completion of the search | Examiner |
|---|---|---|
| Munich | 25 May 2026 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017189176 A

- US 20130166218

**Non-patent literature cited in the description**

- **TAKESHI KAWABATA**. Multiple Sequence Alignment and its Application. *Nara Institute of Science and Technology, Structural and Functional Bioinformatics*, 21 April 2009, http://isw3.naist.jp/IS/Kawabata-lab/LECDOC_KINDAI/2009/multi_09Apr21.pdf>
- Bioinformatics (2) Basic Sequence Analysis. **TATSUYA AKUTSU**. Bioinformatics Center, Institute for Chemical Research. Kyoto University, 2019

- **TTH NGUYEN et al.** Survey of Post-OCR Processing Approaches. *ACM Computing Surveys*, July 2021, vol. 54 (6)
- **TAKAMITSU MATSUBARA** ; **JUN MORIMOTO**. Canonical Multiple Sequence Alignment for Multiple Time-Series Analysis. *IEICE Transactions*, 2013, vol. J96-D (2), 298-305